# EUROPÄISCHE PATENTANMELDUNG

(11) **EP 1 097 914 A2**
(43) Veröffentlichungstag der Anmeldung: **09.05.2001**
(21) Anmeldenummer: 00123094.5
(22) Anmeldetag: 25.10.2000
(51) Int. Cl.: C07B 39/00, C07C 201/16

(54) **Verfahren zur Aufarbeitung von Reaktionsgemischen, hergestellt durch Halexreaktion**

(30) Priorität: 05.11.1999 DE 19953194
(71) Anmelder: Clariant GmbH, 65929 Frankfurt am Main (DE)
(72) Erfinder: Schach, Thomas, Dr., 67294 Gauersheim (DE); Papenfuhs, Theodor, Dr., 60433 Frankfurt (DE); Kaus, Andreas, 65439 Flörsheim (DE); Gutermuth, Maren, 64653 Lorsch (DE)

(57) **Zusammenfassung**

Die vorliegende Erfindung betrifft ein Verfahren zur Aufarbeitung von Verbindungen der Formel (1)

AZₓArF_{w}Cl_{(y-w)}R_{z} (1)

enthaltenen Reaktionsgemischen,
worin in Formel (1) Az unabhängig voneinander gleich oder verschieden ist und für einen Rest -F, -Cl, -Br, -NO₂, -CN, -CF₃, -CCl₃, -CHO, -CO(CₙH₂ₙ₊₁), wobei n eine ganze Zahl von 1 bis 10 ist, -COX oder -SO₂X steht, wobei X für F, Cl oder Br steht, x eine ganze Zahl von 1 bis 3 ist, Ar einen Phenyl-Rest, Pyridyl-Rest oder Naphthylrest bedeutet, w eine ganze Zahl von 1 bis y ist, y eine ganze Zahl von 1 bis 5 bedeutet, R unabhängig voneinander gleich oder verschieden ist und für H, einen geradkettigen oder verzweigten Alkylrest mit 1 bis 10 Kohlenstoffatomen oder einen geradkettigen oder verzweigten Alkoxyrest mit 1 bis 10 Kohlenstoffatomen steht, z eine ganze Zahl von 1 bis 5 ist, wobei (x+y+z) die Zahl aller substituierbarer Valenzen am Rest Ar bedeutet,
indem man ein durch Umsetzung einer Verbindung der Formel (2)

AzₓArCl_{y}R_{z} (2)

mit einem Alkalimetallfluorid oder einem Gemisch von Alkalimetallfluoriden in Gegenwart einer Komponente a) oder einer Mischung der Komponente a) und wenigstens einer der Komponenten b), c), d) und e) als Katalysator in Anwesenheit oder Abwesenheit eines organischen Lösungsmittels bei 50 bis 250°C hergestelltes Reaktionsgemisch mit Wasser versetzt, Reaktionsgemisch und Wasser in innigen Kontakt bringt und die wäßrige Phase abtrennt,
worin in Formel (2) Az, x, Ar, y, R, z und (x + y + z) die gleiche Bedeutung wie in Formel (1) haben
und wobei Komponente a) eine oder mehrere quartäre Ammoniumverbindungen, die einen oder mehrere Reste -(CₘH₂ₘO)ₚR⁵ enthalten, bedeutet, Komponente b) ein oder mehrere Amidophosphoniumsalze, Komponente c) eine oder mehrere quartäre Ammoniumverbindungen, Komponente d) eine oder mehrere quartäre Phosphoniumverbindungen und Komponente e) einen oder mehrere Ether bedeutet.

## Beschreibung

Die vorliegende Erfindung betrifft ein vorteilhaftes Verfahren zur Aufarbeitung von Reaktionsgemischen, die durch Halex-Reaktion (Chlor-/Fluor-Austauschreaktionen) hergestellt werden.

Fluorierte aromatische Verbindungen, insbesondere Fluorbenzole bzw. Fluorbenzolderivate, spielen eine bedeutende Rolle als Zwischenprodukte für die Herstellung von Pflanzenschutzmitteln (Herbiziden) und dienen als Synthesebausteine für Pharmazeutika, beispielsweise zur Herstellung von Chinoloncarbonsäuren und lassen sich auch als Vorprodukte zur Herstellung von Farbstoffen verwenden.

Der Austausch eines Halogens, vorzugsweise von Chlor oder Brom in aktivierten Chlor- oder Bromaromaten, gegen Fluor stellt eine günstige Methode dar, Fluorsubstituenten in ein aromatisches System einzuführen. Im allgemeinen wird diese Reaktion in Gegenwart von aprotisch dipolaren Lösungsmitteln, die in vergleichsweise hohen Mengen eingesetzt werden, und Alkalimetallfluoriden als Fluoridquelle durchgeführt (US 4,226,811).

In neuerer Zeit sind auch lösungsmittelfreie Verfahren bekannt geworden. Diese lösungsmittelfreien Verfahren werden in Gegenwart von Phasentransfer-Katalysatoren durchgeführt. Als besonders vorteilhaft haben sich als Katalysatoren beispielsweise quartäre Ammoniumverbindungen oder quartäre Phosphoniumverbindungen erwiesen.

Die EP 0 908 443 betrifft ein Verfahren zur Herstellung von fluorierten aromatischen Verbindungen der allgemeinen Formel (1) AzₓArF_{w}Cl_{(y-w)}R_{z} durch Umsetzung einer Verbindung der allgemeinen Formel (2) AzₓArCl_{y}R_{z} mit einem Alkalimetallfluorid oder Alkalimetallfluoridgemisch in Gegenwart einer Komponente a) oder einer Mischung der Komponente a) mit wenigstens einer der Komponenten b), c), d) und e), wobei a) eine oder mehrere quartäre Ammoniumverbindungen, die einen oder mehrere geradkettige oder verzweigte Reste -(CₘH₂ₘO)ₚR⁵ besitzen, b) ein oder mehrere Amidophosphoniumsalze, c) eine oder mehrere quartäre Ammoniumverbindungen, d) ein oder mehrere quartäre Phosphoniumverbindungen und e) einen oder mehrere Ether respektive Polyether oder Kronenether bedeutet, unter destillativer Abtrennung der Verbindung (1) und Zugabe der Verbindung (2) zu der Reaktionsmischung.

Der in der EP 0 908 448 Alkoxypolyoxyalkylrest genannte Rest -(CₘH₂ₘO)ₚR⁵ wird nachfolgend präziser als Alkylpolyoxyalkylrest bezeichnet.

Die Formeln (1) und (2) sowie die Komponenten a), b), c), d) und e) haben dieselbe Bedeutung wie nachstehend genauer erläutert wird.

An die vorstehend beschriebenen Umsetzungen, die zu Alkalimetallfluoride und/oder Alkalimetallchloride enthaltenden Reaktionsgemischen führen, schließt sich ein weitgehend übereinstimmender Aufarbeitungsprozess an. Hierbei werden aus den am Ende der Umsetzung erhaltenen Reaktionsgemischen die ausgefallenen Salze durch Filtration abgetrennt. Die abgetrennten Salze werden mit einem organischen Lösungsmittel gewaschen. Die durch Filtration abgetrennte organische Phase wird mit der bei der Wäsche der Salze erhaltenen organischen Lösung in der Regel vereinigt und üblicherweise durch eine Dünnschichtdestillation und/oder eine Fallfilmverdampferdestillation von leicht zersetzlichen Nebenprodukten abgetrennt und nachfolgend fraktioniert destilliert.

Auch die abgetrennten Salze müssen nach der Wäsche mit dem organischen Lösungsmittel von anhaftenden Produktrückständen und/oder Lösemittelresten, beispielsweise in einem Trockner, befreit werden, bevor sie entsorgt oder weiterverarbeitet werden können.

Dieser allgemein übliche Aufarbeitungsprozess erfordert eine Reihe arbeitsintensiver und zeitraubender Aufarbeitungsstufen, die mit einem erheblichen apparativen Aufwand verbunden sind.

Im Hinblick hierauf stellt sich die Aufgabe, ein Verfahren bereitzustellen, das die vorstehend beschriebenen Nachteile nicht aufweist und sich auf einfache Weise mit einem vertretbaren technischen Aufwand realisieren läßt.

Gelöst wird diese Aufgabe durch ein Verfahren zur Aufarbeitung von Verbindungen der Formel (1)

AzₓArF_{w}Cl_{(y-w)}R_{z} (1)

enthaltenen Reaktionsgemischen,
worin in Formel (1) Az unabhängig voneinander gleich oder verschieden ist und für einen Rest -F, -Cl, -Br, -NO₂, -CN, -CF₃, -CCl₃, -CHO, -CO(CₙH₂ₙ₊₁), wobei n eine ganze Zahl von 1 bis 10 ist, -COX oder -SO₂X steht, wobei X für F, Cl oder Br steht, x eine ganze Zahl von 1 bis 3 ist, Ar einen Phenyl-Rest, Pyridyl-Rest oder Naphthylrest bedeutet, w eine ganze Zahl von 1 bis y ist, y eine ganze Zahl von 1 bis 5 bedeutet, R unabhängig voneinander gleich oder verschieden ist und für H, einen geradkettigen oder verzweigten Alkylrest mit 1 bis 10 Kohlenstoffatomen oder einen geradkettigen oder verzweigten Alkoxyrest mit 1 bis 10 Kohlenstoffatomen steht, z eine ganze Zahl von 1 bis 5 ist, wobei (x+y+z) die Zahl aller substituierbarer Valenzen am Rest Ar bedeutet.
Es ist dadurch gekennzeichnet, daß man ein durch Umsetzung einer Verbindung der Formel (2)

AzₓArCl_{y}R_{z} (2)

mit einem Alkalimetallfluorid oder einem Gemisch von Alkalimetallfluoriden in Gegenwart einer Komponente a) oder einer Mischung der Komponente a) und wenigstens einer der Komponenten b), c), d) und e) als Katalysator in Anwesenheit oder Abwesenheit eines organischen Lösungsmittels bei 50 bis 250°C hergestelltes Reaktionsgemisch mit Wasser versetzt, Reaktionsgemisch und Wasser in innigen Kontakt bringt und die wäßrige Phase abtrennt,
worin in Formel (2) Az, x, Ar, y, R, z und (x + y + z) die gleiche Bedeutung wie in Formel (1) haben
und wobei Komponente a) eine oder mehrere quartäre Ammoniumverbindungen der Formel (3) 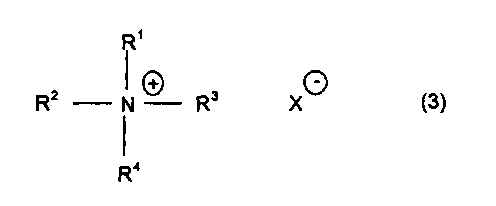 bedeutet,
worin
- R¹, R² und R³: gleich oder verschieden sind und einen geradkettigen oder verzweigten Rest der Formel -(CₘH₂ₘO)ₚR⁵ bedeuten, worin R⁵ für Wasserstoff oder einen geradkettigen oder verzweigten Alkylrest mit 1 bis 16 Kohlenstoffatomen steht, m eine ganze Zahl von 1 bis 10 und p eine Zahl von 1 bis 15 bedeuten;
oder einen geradkettigen oder verzweigten Alkylrest mit 1 bis 30 Kohlenstoffatomen;
oder einen unsubstituierten Phenyl- oder Naphthylrest; oder einen substituierten Phenyl- oder Naphthylrest bedeuten, wobei die Substituenten die Bedeutung Halogen, C₁-C₄-Alkyl, C₁-C₄-Alkoxy, Nitro oder Cyano haben;
- R⁴: einen geradkettigen oder verzweigten Rest der Formel -(CₘH₂ₘO)ₚR⁵ bedeutet; und
- X^{e}: ein einwertiger Säurerest oder das Äquivalent eines mehrwertigen Säurerests ist,
die Komponente b) ein oder mehrere Amidophosphoniumsalze der Formel (4) 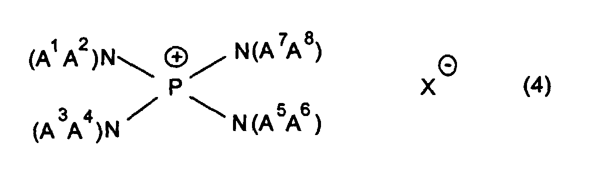 bedeutet,
worin A¹, A², A³, A⁴, A⁵, A⁶, A⁷, A⁸ unabhängig voneinander, gleich oder verschieden sind und für ein geradkettiges oder verzweigtes Alkyl oder Alkenyl mit 1 bis 12 Kohlenstoffatomen, Cycloalkyl mit 4 bis 8 Kohlenstoffatomen, für ein Aryl mit 6 bis 12 Kohlenstoffatomen, ein Aralkyl mit 7 bis 12 Kohlenstoffatomen stehen, oder A¹A², A³A⁴, A⁵A⁶, A⁷A⁸ unabhängig voneinander, gleich oder verschieden sind und direkt oder über O oder N-A⁹ miteinander zu einem Ring mit 3 bis 7 Ringgliedern verbunden sind, A⁹ für ein Alkyl mit 1 bis 4 Kohlenstoffatomen und X⁻ ein einwertiger Säurerest oder das Äquivalent eines mehrwertigen Säurerestes ist, die Komponente c) eine oder mehrere quartäre Ammoniumverbindungen der Formel (5) 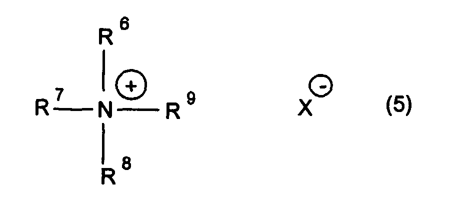 bedeutet,
worin
- R⁶, R⁷, R⁸ und R⁹: gleich oder verschieden sind und für einen geradkettigen oder verzweigten Alkylrest mit 1 bis 22 Kohlenstoffatomen; oder für einen unsubstituierten oder substituierten Arylrest oder für einen C₁-C₄-Alkyl-arylrest stehen, wobei Aryl die Bedeutung Phenyl oder Naphthyl hat und die besagten Substituenten Halogen, C₁-C₄-Alkyl, C₁-C₄-Alkoxy, Nitro oder Cyano bedeuten; und X⁻ ein einwertiger Säurerest oder das Äquivalent eines mehrwertigen Säurerests ist;
die Komponente d) eine oder mehrere quartäre Phosphoniumverbindungen der Formel (6) 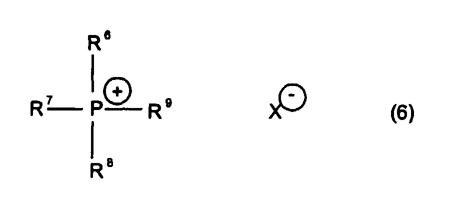 bedeutet,
worin
- R⁶, R⁷, R⁸ und R⁹: gleich oder verschieden sind und für einen linearen oder verzweigten Alkylrest mit 1 bis 22 Kohlenstoffatomen; oder für einen unsubstituierten oder substituierten Arylrest oder für einen C₁-C₄-Alkyl-arylrest stehen, wobei Aryl die Bedeutung Phenyl oder Naphthyl hat und die besagten Substituenten Halogen, C₁-C₄-Alkyl, C₁-C₄-Alkoxy, Nitro oder Cyano bedeuten; und X⁻ ein einwertiger Säurerest oder das Äquivalent eines mehrwertigen Säurerests ist;
die Komponente e) einen oder mehrere Ether der Formel (7)

R¹⁰-(O-CₐH₂ₐ)_{b}-OR¹¹ (7)

oder Kronenether bedeutet, wobei in Formel (7)
- R¹⁰ und R¹¹: gleich oder verschieden sind und einen geradkettigen oder verzweigten Alkylrest mit 1 bis 16 Kohlenstoffatomen bedeuten;
- a: eine ganze Zahl von 2 bis 6 und
- b: eine ganze Zahl von 0 bis 20 ist.

Die vorstehend beschriebene aufwendige Aufarbeitung der Reaktionsgemische läßt sich erfindungsgemäß in überraschender Weise deutlich vereinfachen. Statt der Abtrennung der Salze durch Filtration, Wäsche der Salze mit einem organischen Lösungsmittel, Trocknung der Salze, Vereinigung der organischen Phasen, Dünnschichtdestillation und fraktionierter Destillation wird das Reaktionsgemisch nach der Umsetzung mit Wasser versetzt, intensiv vermischt und anschließend die wässrige Phase von der organischen, das Wertprodukt (Verbindung der Formel (1)) enthaltenden Phase abgetrennt und die organische Phase fraktioniert destilliert. Die Dünnschichtdestillation ist nur noch im Bedarfsfall durchzuführen. Auf sie kann in einer Reihe von Fällen verzichtet werden. In diesen Fällen werden vermutlich thermisch labile Zersetzungspunkte, beispielsweise Nitrophenolate, extrahiert und mit der wässrigen Phase abgetrennt. Die zurückbleibende organische Phase wird dadurch thermisch stabiler, so dass eine Dünnschichtdestillation entfallen kann. Statt der Filtration, Wäsche der abfiltrierten Salze und Trocknung der gewaschenen Satze ist lediglich nach Zugabe von Wasser und Vermischen eine Phasentrennung erforderlich.
Ein weiterer Vorteil ergibt sich dadurch, dass die getrockneten Salze nicht mehr in Transportbehälter abgefüllt werden müssen. Dadurch werden Probleme, die sich bei Bildung von Stäuben ergeben und die Handhabung trockener Salze erschweren, grundsätzlich vermieden.

Die weitere Behandlung der Salze vereinfacht sich in vorteilhafter Weise, da die anfallenden wässrigen Salzlösungen, zum Beispiel zur Rückgewinnung von Kalium, direkt weiterverarbeitet werden können.

Es ist weiterhin als überraschend anzusehen, dass das erfindungsgemäße Verfahren - im Vergleich zu der zuvor beschriebenen üblichen Aufarbeitung - nicht zu einer Verringerung der Ausbeute, sondern in einer Vielzahl von Fällen sogar zu einer Erhöhung der Ausbeute führt Dies war nicht zu erwarten und stellt einen zusätzlichen Vorteil dar.

Nachfolgend soll der Vollständigkeit halber näher auf die Umsetzung der Verbindung der Formel (2) eingegangen werden.

Man setzt als Katalysator üblicherweise die Komponente a) oder die Mischung der Komponente a) mit wenigstens einer der Komponenten b), c), d) und/oder e) in einer Menge von 1 bis 40, insbesondere 2 bis 30, bevorzugt 5 bis 25, besonders bevorzugt 8 bis 20 Gew.-%, bezogen auf die Verbindung der Formel (2) ein. In einer Vielzahl von Fällen kann man die Umsetzung in Gegenwart der Komponente a) oder der Mischung von Komponente a) und wenigstens einer der Komponenten b) und d) durchführen. In diesem Falle fungiert entweder die Komponente a) oder die Mischung von a) + b) oder a) + d) oder a) + b) + d) als Katalysator.

Man setzt üblicherweise als Komponente a) eine oder mehrere quartäre Ammoniumverbindungen der Formel (3) ein, worin R¹, R² und R³ gleich oder verschieden sind und einen linearen (geradkettigen) oder verzweigten Alkylpolyoxyalkyl-Rest der Formel -(CₘH₂ₘO)ₚR⁵ bedeuten, worin R⁵ für Wasserstoff oder einen geradkettigen oder verzweigten Alkylrest mit 1 bis 8 Kohlenstoffatomen, insbesondere für einen geradkettigen oder verzweigten Alkylrest mit 1 bis 8, insbesondere 1 bis 4 Kohlenstoffatomen steht, m eine ganze Zahl von 1 bis 5 und p eine ganze Zahl von 2 bis 10 bedeuten, oder einen geradkettigen oder verzweigten Alkylrest mit 1 bis 8 Kohlenstoffatomen; oder einen unsubstituierten Phenyl- oder Naphthylrest bedeuten, R⁴ einen geradkettigen oder verzweigten Rest der Formel - (CₘH₂ₘO)ₚR⁵ bedeutet, worin R⁵ für Wasserstoff oder einen geradkettigen oder verzweigten Alkylrest mit 1 bis 8 Kohlenstoffatomen, insbesondere für einen geradkettigen oder verzweigten Alkylrest mit 1 bis 8, insbesondere 1 bis 4 Kohlenstoffatomen steht, m eine ganze Zahl von 1 bis 5 und p eine ganze Zahl von 2 bis 10 bedeuten und X⁻ Fluorid, Chlorid, Bromid, SO₄²⁻/2 oder Hydrogensulfat ist.

In dem in der Verbindung der Formel (3) enthaltenen geradkettigen oder verzweigten Alkylpolyoxyalkyl-Rest der Formel -(CₘH₂ₘO)ₚR⁵ können gleiche oder unterschiedliche Alkoxy-Einheiten miteinander verknüpft sein.
Die Anzahl der in der Verbindung der Formel (3) enthaltenen geradkettigen oder verzweigten Alkylpolyoxyalkyl-Reste beträgt vorzugsweise 1 oder 2.

Besonders bevorzugte Verbindungen der Formel (3) im Sinne der vorliegenden Erfindung sind Dimethyl-di-(ethylpolyoxypropyl)-ammoniumchlorid, Dimethyl-di-(ethylpolyoxypropylmethylether)-ammoniumchlorid, Dimethyl-(ethylpolyoxypropyl)-(ethylpolyoxypropylmethylether)-ammoniumchlorid, Dimethyl-di-(ethylpolyoxyethyl)ammoniumchlorid, Dimethyl-di-(ethylpolyoxyethylmethylether)-ammoniumchlorid, Dimethyl-(ethylpolyoxyethyl)-(ethylpolyoxyethylmethylether)-ammoniumchlorid, jeweils mit einer mittleren Kettenlänge p von 3, weiterhin Trimethyl-(ethylpolyoxypropyl)-ammoniumchlorid und Trimethyl-(ethylpolyoxypropylmethylether)ammoniumchlorid, jeweils mit einer mittleren Kettenlänge p von 8, oder ein Gemisch der genannten Verbindungen.

Die beschriebenen Verbindungen der Formel (3) lassen sich aus den entsprechenden Ethanolaminen herstellen, die nach Umsetzung mit Alkenyloxiden und anschließender Quaternisierung mit oder ohne gleichzeitiger Veretherung in guten Ausbeuten die gewünschten Verbindungen liefern.
In der Mischung der Komponenten a) und wenigstens einer der Komponenten b), c), d) und/oder e) macht die Komponente a) 5 bis 95, insbesondere 10 bis 90, bevorzugt 20 bis 80 Gew.-% der gesamten Mischung, die als Katalysator eingesetzt wird, aus. Das molare Verhältnis von Katalysator (Komponente a) oder Gemisch von Komponente von a) und wenigstens einer der übrigen Komponenten b) bis e)) zu der Verbindung der Formel (2) kann 1:5 bis 1:150, insbesondere 1:10 bis 1:100, bevorzugt 1:20 bis 1:100 betragen. Man setzt üblicherweise die Verbindung der Formel (2) zu Alkalimetallfluorid in einem molaren Verhältnis von 1:(0,5 bis 3), insbesondere 1:(0,8 bis 2,5), bevorzugt von 1:(0,9 bis 1,5) ein. Diese Angaben beziehen sich auf den Austausch eines Halogens in der Verbindung der Formel (2) gegen Fluor. Sollen zwei oder mehr Halogene in der Verbindung (2) gegen Fluor ausgetauscht werden, so setzt man das Alkalimetallfluorid in entsprechend höherem molaren Verhältnis ein.

### Komponente b):

Man kann eine Verbindung der Formel (4), worin A¹, A², A³, A⁴, A⁵, A⁶, A⁷, A⁸ unabhängig voneinander, gleich oder verschieden sind und für ein geradkettiges oder verzweigtes Alkyl oder Alkenyl, insbesondere Alkyl, mit 1 bis 12, insbesondere 1 bis 8, bevorzugt 1 bis 4 Kohlenstoffatomen, oder Cycloalkyl mit 4 bis 8, insbesondere 5 bis 6 Kohlenstoffatomen, stehen, einsetzen. Diese Verbindungen sind von besonderem Interesse, da sie sich auf vergleichsweise einfache Art ausgehend von den entsprechenden Dialkylaminen, Dialkenylaminen, Dicycloalkylaminen, sekundären Aminen, die einen Alkyl- und Alkenylrest, einen Alkyl- und Cycloalkylrest oder einen Alkenyl- und Cycloalkylrest enthalten, herstellen lassen.

Man kann eine Verbindung der Formel (4), worin A¹A² = A³A⁴ oder A¹A² = A³A⁴ = A⁵A⁶ oder A¹A² = A³A⁴ = A⁵A⁶ = A⁷A⁸ ist, einsetzen. Diese Verbindungen, in denen zwei oder mehrere der Gruppen A¹A², A³A⁴, A⁵A⁶ und A⁷A⁸ einander gleich sind, sind relativ gut zugänglich.

Als Beispiele für Alkyl sind Methyl, Ethyl, n-Propyl, i-Propyl, n-Butyl, i-Butyl, n-Pentyl, 3-Methylbutyl, n-Hexyl, 2-Ethylhexyl, insbesondere Methyl, Ethyl, n-Propyl, n-Butyl und als Beispiele für Alkenyl sind Allyl, Prop-(2)-enyl, n-But-(2)-enyl, und als Beispiele für Cycloalkyl sind Cyclopentyl, Cyclohexyl, 4-Methylcyclohexyl, 4-tert.-Butylcyclohexyl, zu nennen.

Man kann auch eine Verbindung der Formel (4), worin A¹=A², A³=A⁴, A⁵=A⁶ und/oder A⁷=A⁸ ist, einsetzen. Diese Verbindungen sind vergleichsweise leicht zugänglich und deshalb von Interesse.

Man kann auch eine Verbindung der Formel (4), worin A¹ = A² = A³ = A⁴ oder A¹ = A² = A³ = A⁴ = A⁵ = A⁶ oder A¹ = A² = A³ = A⁴ = A⁵ = A⁶ = A⁷ = A⁸ ist, einsetzen. Diese vorstehend genannten Verbindungen, in denen vier, sechs oder acht der Reste A¹ bis A⁸ gleich sind, sind aufgrund ihrer leichten Zugänglichkeit ebenfalls von Interesse.

Es ist auch möglich, eine Verbindung der Formel (4), worin A¹A² oder A¹A² und A³ A⁴ oder A¹A² und A³A⁴ und A⁵A⁶ oder A¹A² und A³A⁴ und A⁵A⁶ und A⁷A⁸ direkt oder über O oder N-A⁹ miteinander zu einem gesättigten oder ungesättigten Ring mit 5 oder 6 Ringgliedern verbunden sind, einzusetzen. Dementsprechend enthalten diese Verbindungen einen, zwei, drei oder vier der vorstehend erwähnten Ringe.

Es ist ferner möglich, eine Verbindung der Formel (4), worin A¹A² oder A¹A² und A³ A⁴ oder A¹A² und A³A⁴ und A⁵A⁶ oder A¹A² und A³A⁴ und A⁵A⁶ und A⁷A⁸ zu einem Ring, der das N-Atom, an dem die jeweiligen Reste A¹ bis A⁸ sitzen, gegebenenfalls O oder N-A⁹ und CH₂-Gruppen als Ringglieder umfaßt, verbunden sind, einzusetzen. In dieser Stoffgruppe bilden das N-Atom mit den an ihnen jeweils befindlichen Resten A¹ bis A⁸ beispielsweise einen Hexahydropyridinring (Piperidinring), Tetrahydropyrrolring (Pyrrolidinring), einen Hexahydropyrazinring (Piperazinring) oder Morpholinring.
Dementsprechend enthalten diese Verbindungen einen, zwei, drei oder vier der vorstehend erwähnten Ringe.

In den Verbindungen der Formeln (3), (4), (5) und (6) steht X⁻, wie bereits eingangs erwähnt, für einen einwertigen Säurerest oder das Äquivalent eines mehrwertigen Säurerestes, insbesondere den Rest einer anorganischen Mineralsäure, einer organischen Carbonsäure, einer aliphatischen oder aromatischen Sulfonsäure.

Üblicherweise setzt man eine Verbindung der Formel (3), (4), (5) und (6) worin X⁻ für F⁻, Cl⁻, Br⁻, J⁻, HF₂⁻, BF₄⁻, C₆H₅SO₃⁻, p-CH₃-C₆H₅SO₃⁻, SO₄²⁻/2, HSO₄⁻, PF₆⁻, CF₃SO₃⁻, insbesondere für F⁻, Cl⁻, BR⁻, J⁻, HF₂⁻, BF₄⁻, steht, ein.

Als Komponente b) kann man ein oder mehrere Amidophosphoniumsalze der Formel (4), worin drei Reste (A¹A²)N, (A³A⁴)N und (A⁵A⁶)N gleich sind und für einen Dialkylaminorest mit 1 bis 6, insbesondere 1 bis 4 Kohlenstoffatomen je Alkyl, der Dialkylaminorest insbesondere zwei gleiche Alkylgruppen enthält, oder für einen Pyrrolidin-, Piperidin- oder Morpholinring stehen, der Rest (A^{⁷}A⁸)N sich von den vorstehend genannten Resten unterscheidet, wobei A⁷ und A⁸ gleich oder verschieden sind und für einen Alkylrest mit 1 bis 8 Kohlenstoffatomen oder einen Alkenylrest mit 1 bis 4 Kohlenstoffatomen stehen, oder alle vier Reste (A¹A²)N, (A³A⁴)N, (A⁵A⁶)N und (A^{⁷}A⁸)N gleich sind und für einen Dialkylaminorest mit 1 bis 6, insbesondere 1 bis 4, bevorzugt 1 bis 2 Kohlenstoffatomen je Alkyl oder für einen Pyrolidin-, Piperidin- oder Morpholin-Ring stehen, einsetzen.

Ohne Anspruch auf Vollständigkeit zu erheben, seien als Beispiele für Verbindungen der Formel (4) genannt:
Tetrakis(dimethylamino)phosphoniumchlorid
Tetrakis(diethylamino)phosphoniumchlorid
Tetrakis(dimethylamino)phosphoniumbromid
Tetrakis(diethylamino)phosphoniumbromid
Tetrakis(dipropylamino)phosphoniumchlorid oder -bromid
Tris(diethylamino)(dimethylamino)phosphoniumchlorid oder -bromid
Tetrakis(dibutylamino)phosphoniumchlorid oder -bromid
Tris(dimethylamino)(diethylamino)phosphoniumchlorid oder -bromid
Tris(dimethylamino)(cyclopentylamino)phosphoniumchlorid oder -bromid
Tris(dimethylamino)(dipropylamino)phosphoniumchlorid oder -bromid
Tris(dimethylamino)(dibutylamino)phosphoniumchlorid oder -bromid
Tris(dimethylamino)(cyclohexylamino)phosphoniumchlorid oder -bromid
Tris(dimethylamino)(diallylamino)phosphoniumchlorid oder -bromid
Tris(dimethylamino)(dihexylamino)phosphoniumchlorid oder -bromid
Tris(diethylamino)(dihexylamino)phosphoniumchlorid oder -bromid
Tris(dimethylamino)(diheptylamino)phosphoniumchlorid oder -bromid
Tris(diethylamino)(diheptylamino)phosphoniumchlorid oder -bromid
Tetrakis(pyrrolidino)phosphoniumchlorid oder -bromid
Tetrakis(piperidino)phosphoniumchlorid oder -bromid
Tetrakis(morpholino)phosphoniumchlorid oder -bromid
Tris(piperidino)(diallylamino)phosphoniumchlorid oder -bromid
Tris(pyrrolidino)(ethylmethylamino)phosphoniumchlorid oder -bromid
Tris(pyrrolidino)(diethylamino)phosphoniumchlorid oder -bromid.

Man kann auch ein Gemisch zweier oder mehrerer Verbindungen der Formel (4) verwenden. Besonders einfach gestaltet sich dies, wenn man Gemische von Verbindungen der Formel (4) verwendet.

Es hat sich bewährt, als Komponente c) ein oder mehrere quartäre Ammoniumverbindungen der Formel (5), worin R⁶, R⁷ und R⁸ gleich sind und für einen Alkylrest mit 1 bis 4, insbesondere 1 bis 2 Kohlenstoffatomen stehen und R⁹ ein Alkylrest mit 6 bis 24, insbesondere 8 bis 20, bevorzugt 10 bis 18 Kohlenstoffatomen ist oder R⁶, R⁷, R⁸ und R⁹ gleich sind und einen Alkylrest mit 1 bis 6, insbesondere 1 bis 4 Kohlenstoffatomen bedeuten, einsetzt.

Ohne Anspruch auf Vollständigkeit zu erheben, seien als einige Vertreter der Komponente c) Octadecyltrimethylammoniumchlorid, Hexadecyltrimethylammoniumchlorid, Benzyltrimethylammoniumchlorid, Tetramethylammoniumchlorid, Tetramethylammoniumbromid, Tetraethylammoniumchlorid , Tetraethylammoniumbromid, Tetrabutylammoniumchlorid und Tetrabutylammoniumbromid genannt. Es lassen sich auch Gemische der vorstehenden Verbindungen verwenden.

Es hat sich ferner als günstig erwiesen, als Komponente d) eine oder mehrere quartäre Phosphoniumverbindungen der Formel (6), worin R⁶, R⁷ und R⁸ gleich sind und für einen Alkylrest mit 1 bis 4, insbesondere 1 bis 2 Kohlenstoffatomen stehen und R⁹ ein Alkylrest mit 6 bis 24, insbesondere 8 bis 20, bevorzugt 10 bis 18 Kohlenstoffatomen ist, oder R⁶, R⁷, R⁸ und R⁹ gleich sind und für einen Phenylrest oder einen Alkylrest mit 1 bis 6, insbesondere 1 bis 4 Kohlenstoffatomen bedeuten, einzusetzen.

Als Beispiele für geeignete Verbindungen der Komponente d) seien Hexadecyltributylphosphoniumbromid, Stearyltributylphosphoniumbromid, Tetrabutylphosphoniumchlorid, Tetrabutylphosphoniumbromid, Tetraoctylphosphoniumbromid, Tetraphenylphosphoniumchlorid und Tetraphenylphosphoniumbromid erwähnt. Es lassen sich auch Gemische der vorstehenden Verbindungen verwenden.

In einer Reihe von Fällen ist es von Vorteil, als Komponente e) einen oder mehrere Ether respektive Polyether der Formel (7), worin R¹⁰ und R¹¹ gleich oder verschieden sind und einen geradkettigen oder verzweigten Alkylrest mit 1 bis 8, insbesondere 1 bis 6 Kohlenstoffatomen bedeuten, a eine ganze Zahl von 2 bis 3 und b eine ganze Zahl von 4 bis 14, insbesondere 4 bis 8 ist, oder einen Kronenether, insbesondere einen Polyether der Formel (7), worin R¹⁰ und R¹¹ gleich sind und einen Alkylrest mit 1 bis 8, insbesondere 1 bis 6 Kohlenstoffatomen bedeuten, a eine ganze Zahl von 2 bis 3 und b eine ganze Zahl von 4 bis 14, insbesondere 6 bis 10 ist, einzusetzen.

Von Interesse sind Gemische der Komponente a), b), c), d) und e) wenn a):b) im Gewichtsverhältnis 10:1 bis 1:10, insbesondere 5:1 bis 1:5 und die Komponenten c), d) und e) in einer Menge von 0 bis 25, insbesondere 5 bis 20 Gew.-%, bezogen auf die Mischung enthalten sind.

In einer Vielzahl von Fällen hat sich eine Mischung der Komponenten a), b) und d), worin a): b): d) im Gewichtsverhältnis (10 bis 1):(5 bis 0,1): (5 bis 0,1), insbesondere (8 bis 2) : (3 bis 0,5) : (3 bis 0,5) enthalten sind, als günstig erwiesen.

Mischungen der Komponenten a) und d), worin a) : d) im Gewichtsverhältnis 10:1 bis 1:10, insbesondere 5:1 bis 1:5 enthalten sind, lassen sich auch mit Erfolg einsetzen.

Man führt die Umsetzung der Verbindung der Formel (2), wie eingangs bereits erwähnt, bei 50 bis 250, insbesondere 70 bis 220, bevorzugt 100 bis 200°C durch.

Es stellt einen weiteren Vorteil dar, daß man die Umsetzung der Verbindung der Formel (2) mit dem Alkalimetallfluorid in Abwesenheit eines Lösungsmittels, insbesondere in Abwesenheit eines dipolar aprotischen Lösungsmittels durchführen kann. Beispiele für dipolar aprotische Lösungsmittel sind Dimethylsulfoxid, Dimethylformamid, Diethylformamid, Dimethylacetamid, Diethylacetamid, Hexamethylphosphorsäuretriamid, Tetramethylharnstoff, Sulfolan (Tetramethylensulfon) und N-Methylpyrrolidon. Das erfindungsgemäße Verfahren eröffnet in vorteilhafter Weise einen Weg, auf die Verwendung solcher Lösungsmittel, die üblicherweise in großen Mengen, bezogen auf das Ausgangsmaterial, eingesetzt werden, zu verzichten. Dadurch kann das für die Umsetzung zur Verfügung stehende Reaktorvolumen wesentlich besser ausgenutzt werden.

Falls man in Anwesenheit eines Lösungsmittels die Umsetzung durchführen will, setzt man üblicherweise ein Lösungsmittel, vorzugsweise in untergeordneten Mengen, beispielsweise in einer Menge von 0,5 bis 10, insbesondere 1 bis 3 Gew.-%, bezogen auf Reaktionsmischung ein. Beispiele für geeignete Lösungsmittel sind, ohne Anspruch auf Vollständigkeit zu erheben, Toluol, Chlortoluol, Chlorbenzol, Dichlorbenzol, Ethylbenzol, o-Xylol, m-Xylol, p-Xylol, technische Gemische isomerer Xylole und/oder eines oder mehrere der vorstehend genannten dipolar aprotischen Lösungsmittel.

Man kann mit gutem Erfolg eine Verbindung der Formel (2), worin Az unabhängig voneinander gleich oder verschieden ist und für einen Rest -F, -Cl, -NO₂, -CN, -CF₃, -CCl₃, -CHO oder -CO(CₙH₂ₙ₊₁) steht, wobei n eine ganze Zahl von 1 bis 6, insbesondere 1 bis 4, vorzugsweise 1 bis 2 ist, insbesondere für einen Rest -Cl, -NO₂, -CN, -CF₃ oder -CHO steht, umsetzen.

Man kann insbesondere eine Verbindung der Formel (2), worin Ar für einen Phenyl-Rest oder Pyridyl-Rest, insbesondere für einen Phenyl-Rest steht, als geeignete Ausgangsverbindung umsetzen.

In der Verbindung der Formel (2) steht y, wie zuvor erwähnt, für eine ganze Zahl von 1 bis 5, insbesondere 1 bis 4, bevorzugt 1 bis 3, besonders bevorzugt 1 bis 2.

Man kann eine Verbindung der Formel (2) umsetzen, worin wie zuvor bereits aufgeführt, R unabhängig voneinander gleich oder verschieden ist und für H, einen geradkettigen oder verzweigten Alkylrest mit 1 bis 10 Kohlenstoffatomen oder einen geradkettigen oder verzweigten Alkoxyrest mit 1 bis 10 Kohlenstoffatomen, insbesondere für H, einen geradkettigen oder verzweigten Alkylrest mit 1 bis 4 Kohlenstoffatomen oder einen geradkettigen oder verzweigten Alkoxyrest mit 1 bis 4 Kohlenstoffatomen, bevorzugt für H, einen Alkyl- oder Alkoxyrest mit 1 bis 2 Kohlenstoffatomen, besonders bevorzugt für H steht.

Man setzt als Alkalimetallfluorid NaF, KF, RbF, CsF oder ein Gemisch derselben, insbesondere KF oder ein Gemisch aus KF, RbF und/oder CsF ein.

Für eine Vielzahl von Fällen hat es sich als ausreichend erwiesen, als Alkalimetalllfluorid KF zu verwenden. In einigen Fällen haben sich Gemische aus KF und CsF, die 1 bis 10 Gew.-% CsF enthalten, als vorteilhaft gezeigt.

Durch die vorstehend näher beschriebene Umsetzung der Verbindung der Formel (2) mit dem Alkalimetallfluorid in Gegenwart der Komponente a) oder einer Mischung der Komponente a) und wenigstens einer der Komponenten b), c), d) und e) als Katalysator gelangt man zu einem Reaktionsgemisch, das entweder unmittelbar, das heißt, ohne destillative Abtrennung niedrig siedender Komponenten oder nach destillativer Abtrennung niedrig siedender Komponenten in das erfindungsgemäße Verfahren zur Aufarbeitung von Verbindungen der Formel (1) enthaltenden Reaktionsgemischen eingesetzt wird.

Das Reaktionsgemisch wird - wie zuvor bereits erwähnt - mit Wasser versetzt. Reaktionsgemisch und Wasser werden in innigen Kontakt gebracht und die wässrige Phase wird abgetrennt. Man bringt, beispielsweise durch intensives Rühren, Umpumpen, mechanisches Vermischen oder vergleichbare Maßnahmen, Reaktionsgemisch und Wasser in innigen Kontakt. Auf diese Weise wird sichergestellt, daß die wasserlöslichen Bestandteile des Reaktionsgemisches aus dem Reaktionsgemisch herausgelöst werden und in die wässrige Phase gelangen.

Zu den wasserlöslichen Bestandteilen des Reaktionsgemisches gehören Alkalimetallsalze, insbesondere Alkalimetallhalogenide. Infolge der Umsetzung der Verbindung der Formel (2) bildet sich aufgrund des Chlor-Fluor-Austauschs aus dem Alkalimetallfluorid das entsprechende Alkalimetallchlorid. Üblicherweise enthält das Reaktionsgemisch ein Gemisch aus nicht umgesetzten Alkalimetallfluoriden und Alkalimetallchloriden. Bei Einsatz beispielsweise von KF als Alkalimetallfluorid liegt im Reaktionsgemisch KCl als Alkalimetallchlorid neben gegebenenfalls nicht umgesetztem KF vor. Setzt man Mischungen von Alkalimetallfluoriden ein, so entstehen entsprechende Mischungen von Alkalimetallchloriden, die gegebenenfalls nicht umgesetzte Alkalimetallfluoride enthalten.

Zu den wasserlöslichen Bestandteilen der Reaktionsmischungen gehören auch die wasserlöslichen Bestandteile des Katalysators, beispielsweise die quartären Ammoniumverbindungen der Formel (3), die Amidophosphoniumsalze der Formel (4), die quartären Ammoniumverbindungen der Formel (5), die quartären Phosphoniumverbindungen der Formel (6), die Polyether der Formel (7) und Kronenether, also die Komponente a), b), c), d) und e). Die Katalysatorkomponenten gehen in der Regel überwiegend oder insgesamt in die wässrige Phase über.

Man versetzt das Reaktionsgemisch üblicherweise bei 20 bis 80°C, insbesondere 20 bis 50°C mit Wasser, bringt Reaktionsgemisch und Wasser in Kontakt und trennt die wässrige Phase ab.

Es ist auch möglich, das Reaktionsgemisch zunächst mit Wasser zu versetzen und das Reaktionsgemisch mit Wasser anschließend bei 20 bis 80°C, insbesondere 20 bis 50°C in Kontakt zu bringen, indem man die gewünschte Temperatur nach Zugabe des Wassers während der Extraktion einstellt und anschließend die wässrige Phase abtrennt.

Man kann auch bei tieferen Temperaturen, beispielsweise bei 10°C wie vorstehend beschrieben arbeiten, muß dann allerdings eine geringere Wasserlöslichkeit der abzutrennenden wasserlöslichen Bestandteile in Kauf nehmen.

Niedrige Temperaturen wirken sich günstig hinsichtlich der Korrosivität des Reaktionsgemisches und der wässrigen Phase aus und verringern unerwünschte Nebenreaktionen. Bei niedrigen Temperaturen ist die Korrosion an üblichen Reaktormaterialien gering.

Liegt der Erstarrungspunkt des Reaktionsgemisches oder einzelner Stoffe (Einsatz- und/oder Endprodukte) über dem vorstehend genannten Temperaturbereich, so können auch entsprechend höhere Temperaturen oberhalb des Erstarrungspunktes angewendet werden.

Es hat sich üblicherweise als ausreichend erwiesen, das Reaktionsgemisch und Wasser im Gewichtsverhältnis 1: (0,1 bis 10), insbesondere 1: (0,5 bis 5), bevorzugt 1: (1 bis 3) einzusetzen.

Man versetzt das Reaktionsgemisch üblicherweise mit einer Menge Wasser, die ausreicht, die im Reaktionsgemisch vorhandenen wasserlöslichen Bestandteile zu lösen.

In einer Reihe von Fällen genügt es, das Reaktionsgemisch mit einer Menge Wasser zu versehen, die ausreicht, die im Reaktionsgemisch vorhandenen Alkalimetallsalze, insbesondere Alkalimetallchloride und gegebenenfalls noch vorhandene Alkalimetallfluoride, zu lösen.

Da Alkalimetallfluoride im allgemeinen eine deutlich bessere Wasserlöslichkeit als Alkalimetallchloride besitzen, richtet sich die Wassermenge hauptsächlich nach der im Reaktionsgemisch enthaltenden Menge Alkalichlorid.

Man bringt Reaktionsgemisch und Wasser in Kontakt, indem man Reaktionsgemisch und Wasser intensiv vermischt. Besonders einfach ist es, das Reaktionsgemisch und Wasser durch intensives Rühren zu vermischen. Indem man das Reaktionsgemisch und Wasser in Kontakt bringt, extrahiert man die wasserlöslichen Bestandteile aus dem Reaktionsgemisch und überführt sie in das Wasser. Es bildet sich eine die wasserlöslichen Bestandteile des Reaktionsgemisches enthaltende wässrige Phase, während die organische Phase das Wertprodukt (Verbindung der Formel (1)) enthält.

Am Ende der Extraktion bringt man die resultierenden Phasen - organische Phase und wässrige Phase - nicht mehr in Kontakt, stellt also das Vermischen ein, läßt die beiden Phasen sich absetzen und trennt die wässrige Phase von der organischen Phase ab.

Die organische, das Wertprodukt enthaltende Phase wird anschließend aufgearbeitet. Sollte der durch die Extraktion bedingte Wassergehalt in der organischen Phase stören, so kann das Wasser durch kurzes Andestillieren, gegebenenfalls unter reduziertem Druck, direkt oder durch Azeotropdestillation entfernt werden. Da der Wassergehalt üblicherweise gering ist, genügt ein kurzes Andestillieren.

Enthält die organische Phase leicht zersetzliche Nebenkomponenten, so kann man diese gegebenenfalls durch eine Dünnschichtdestillation abtrennen und anschließend die von den leicht zersetzlichen Produkten befreite organische Phase fraktioniert destillieren. Die fraktionierte Destillation wird unter Normaldruck oder reduziertem Druck, insbesondere unter reduziertem Druck durchgeführt.

Das erfindungsgemäße Verfahren läßt sich kontinuierlich oder diskontinuierlich unter reduziertem Druck, Normaldruck oder erhöhtem Druck durchführen. Es eignet sich besonders für eine diskontinuierliche Arbeitsweise.

Die nachfolgenden Beispiele beschreiben die Erfindung näher, ohne sie zu beschränken.

### Experimenteller Teil

### Beispiel A

### Herstellung eines 5-Chlor-2,4-difluornitrobenzol enthaltenden Reaktionsgemisches

In einem 1500 ml Planschliffkolben, bestückt mit Destillationsbrücke und Ankerrührer, werden 891,0 g (3,54 mol) 2,4,5-Trichlornitrobenzol (roh, 90 %ig) vorgelegt und aufgeschmolzen. In die Schmelze des 2,4,5-Trichlornitrobenzols werden bei 65°C 108,1 g Trimethyl-(ethylpolyoxypropyl)-ammoniumchlorid (89 %ig) und 392,2 g (6,75 mol) Kaliumfluorid unter Rühren eingetragen.
Anschließend gibt man 80 g (0,75 mol) Xylol zu und trocknet das Einsatzgemisch durch Anlegen eines Vakuums von 20 mbar (20 hPa) und durch Erhöhen der Temperatur bis auf 120°C mittels Azeotropdestillation. Sind die 120°C erreicht und destilliert kann Xylol mehr über, wird das Einsatzgemisch 16 Stunden bei 135°C unter intensivem Rühren und unter Atmosphärendruck umgesetzt.

### Beispiel 1

### Aufarbeitung des Reaktionsgemisches unter Zusetzen von Wasser

Die gemäß Beispiel A hergestellte Reaktionsmischung wird nach Beendigung der Umsetzung auf 25 bis 30°C gekühlt und mit 1600 ml Wasser versetzt. Anschließend werden das Reaktionsgemisch und das zugesetzte Wasser unter intensivem Rühren innig in Kontakt gebracht und 15 Minuten kräftig gerührt. Man stellt das Rühren ein und läßt die organische und die wässrige Phase absetzen. Nach Beendigung des Absetzens der Phasen trennt man die untere wässrige Phase, die die Salze und die wasserlöslichen Bestandteile der Reaktionsmischung enthält, von der organischen Phase ab.
Man erhält 2006 g wässrige Phase (pH = 4,5) und 822,4 g organische Phase, die durch fraktionierte Destillation aufgearbeitet wird.
Man erhält 428,6 g (2,22 mol) 5-Chlor-2,4-difluornitrobenzol, entsprechend 62,7 % der Theorie, bezogen auf eingesetztes 2,4,5-Trichlornitrobenzol (90 %ig) und 160,4 g (0,76 mol) Dichlorfluornitrobenzole (monofluorierte Produkte), entsprechend 21,5 % der Theorie, bezogen auf eingesetztes 2,4,5-Trichlornitrobenzol (90 %ig) bzw. 83,2 % der Theorie fluorierte Produkte (5-Chlor-2,4-difluornitrobenzol + Dichlorfluornitrobenzole), bezogen auf eingesetztes 2,4,5-Trichlornitrobenzol (90 %ig).

### Vergleichsbeispiel 1

### Aufarbeitung des Reaktionsgemisches auf herkömmliche Weise

Die gemäß Beispiel A hergestellte Reaktionsmischung wird nach Beendigung der Umsetzung auf 25 bis 30°C gekühlt und die ausgefallenen Salze werden von der organischen Phase durch Filtration über eine Nutsche abgetrennt. Die Salze werden dreimal mit jeweils 100 ml Xylol gewaschen und die Xylollösung wird abgesaugt. Die xylolfeuchten Salze werden von der Nutsche in einen Trockenschrank überführt und bei einem Druck von 30 bis 70 mbar (30 bis 70 hPa) bei 150 bis 160°C 10 bis 16 Stunden von Xylol und anhaftenden organischen Verunreinigungen befreit.

Anschließend wird die organische Phase und die bei der Wäsche der Salze angefallene Xylollösung vereint und fraktioniert destilliert.
Man erhält 398,7 g (2,06 mol) 5-Chlor-2,4-difluornitrobenzol, entsprechend 58,2 % der Theorie, bezogen auf eingesetztes 2,4,5-Trichlornitrobenzol (90 %ig) und 148,1 g (0,71 mol) Dichlorfluornitrobenzole (monofluorierte Produkte), entsprechend 20,1 % der Theorie, bezogen auf eingesetztes 2,4,5-Trichlornitrobenzol (90 %ig) bzw. 78,3 % der Theorie fluorierte Produkte (5-Chlor-2,4-difluornitrobenzol + Dichlorfluornitrobenzole), bezogen auf eingesetztes 2,4,5-Trichlornitrobenzol (90 %ig).

### Beispiel B

### Herstellung eines 2-Fluornitrobenzol enthaltenden Reaktionsgemisches

In einem 2000 ml Planschliffkolben, bestückt mit Destillationsbrücke und Impellerrührer, werden 113,4 g (0,18 mol) Methyl-tris-(methyltetraethoxy)ammoniumchlorid, 56,7 g (0,17 mol) Tetrabutylphosphoniumbromid und 1890 g (12,0 mol) 2-Chlornitrobenzol vorgelegt und aufgeschmolzen. In diese Schmelze werden bei 65°C 697,2 g (12,0 mol) Kaliumfluorid unter Rühren eingetragen.
Anschließend gibt man 1509 (1,4 mol) Xylol zu und trocknet das Einsatzgemisch durch Anlegen eines Vakuums von 20 mbar (20 hPa) und durch Erhöhen der Temperatur bis auf 120°C mittels Azeotropdestillation. Sind die 120°C erreicht und destilliert kein Xylol mehr über, wird das Einsatzgemisch 21 Stunden bei 170°C unter intensivem Rühren und unter Atmosphärendruck umgesetzt.

### Beispiel 2

### Aufarbeitung des Reaktionsgemisches unter Zusetzen von Wasser

Die gemäß Beispiel B hergestellte Reaktionsmischung wird nach Beendigung der Umsetzung auf 25 bis 30°C gekühlt und mit 3000 ml Wasser versetzt. Anschließend werden das Reaktionsgemisch und das zugesetzte Wasser unter intensivem Rühren innig in Kontakt gebracht und 15 Minuten kräftig gerührt. Man stellt das Rühren ein und läßt die organische und die wässrige Phase absetzen. Nach Beendigung des Absetzens der Phasen trennt man die wässrige Phase, die die Salze und die wasserlöslichen Bestandteile der Reaktionsmischung enthält, von der organischen Phase ab.
Man erhält 3850 g wässrige Phase (pH = 4,5) und 1863 g organische Phase, die durch fraktionierte Destillation aufgearbeitet wird.

Man erhält 1150 g (8,2 mol) 2-Fluornitrobenzol, entsprechend 68,3 % der Theorie, bezogen auf eingesetztes 2-Chlornitrobenzol, und 335 g (2,2 mol) nicht umgesetztes 2-Chlornitrobenzol.

### Vergleichsbeispiel 2

### Aufarbeitung des Reaktionsgemisches auf herkömmliche Weise

Die gemäß Beispiel B hergestellte Reaktionsmischung wird nach Beendigung der Umsetzung auf 25 bis 30°C gekühlt und die ausgefallenen Salze werden von der organischen Phase durch Filtration über eine Nutsche abgetrennt. Die Salze werden dreimal mit jeweils 200 ml Xylol gewaschen und die Xylollösung wird abgesaugt. Anschließend arbeitet man wie in Vergleichsbeispiel 1 beschrieben.

Man erhält 1085 g (7,7 mol) 2-Fluornitrobenzol, entsprechend 64,2 % der Theorie, bezogen auf eingesetztes 2-Chlornitrobenzol, und 313 g (2,0 mol) nicht umgesetztes 2-Chlornitrobenzol.

### Beispiel C

### Herstellung eines 2,4-Difluornitrobenzol enthaltenden Reaktionsgemisches

In einem 2000 ml Planschliffkolben, bestückt mit Destillationsbrücke und Impellerrührer, werden 154 g (0,24 mol) Methyl-tris-(methyltetraethoxy)ammoniumchlorid, 46 g (0,13 mol) Tetrabutylphosphoniumbromid und 1534 g (8,0 mol) 2,4-Dichlornitrobenzol vorgelegt und aufgeschmolzen. In diese Schmelze werden bei 65°C 929 g (16,0 mol) Kaliumfluorid unter Rühren eingetragen.
Anschließend gibt man 250 g (2,3 mol) Xylol zu und trocknet das Einsatzgemisch durch Anlegen eines Vakuums von 20 mbar (20 hPa) und durch Erhöhen der Temperatur bis auf 120°C mittels Azeotropdestillation. Sind die 120°C erreicht und destilliert kein Xylol mehr über, wird das Einsatzgemisch 16 Stunden bei 170°C unter intensivem Rühren und Atmosphärendruck umgesetzt.

### Beispiel 3

### Aufarbeitung des Reaktionsgemisches unter Zusetzen von Wasser

Die gemäß Beispiel C hergestellte Reaktionsmischung wird nach Beendigung der Umsetzung auf 25 bis 30°C gekühlt und mit 3900 ml Wasser versetzt. Anschließend werden das Reaktionsgemisch und das zugesetzte Wasser unter intensivem Rühren innig in Kontakt gebracht und 15 Minuten kräftig gerührt. Man stellt das Rühren ein und läßt die organische und die wässrige Phase absetzen. Nach Beendigung des Absetzens der Phasen trennt man die wässrige Phase, die die Salze und die wasserlöslichen Bestandteile der Reaktionsmischung enthält, von der organischen Phase ab.
Man erhält 4900 g wässrige Phase (pH = 4,5) und 1570 g organische Phase, die durch fraktionierte Destillation aufgearbeitet wird. Man erhält 745 g (4,7 mol) 2,4-Difluornitrobenzol, entsprechend 58,8 % der Theorie, bezogen auf eingesetztes 2,4-Dichlornitrobenzol, und insgesamt 339 g (2,1 mol) nichtumgesetztes 2,4-Dichlornitrobenzol + Fluorchlornitrobenzole (monofluorierte Produkte).

### Vergleichsbeispiel 3

### Aufarbeitung des Reaktionsgemisches auf herkömmliche Weise

Die gemäß Beispiel C hergestellte Reaktionsmischung wird nach Beendigung der Umsetzung auf 25 bis 30°C gekühlt und die ausgefallenen Salze werden von der organischen Phase durch Filtration über eine Nutsche abgetrennt. Die Salze werden dreimal mit jeweils 200 ml Xylol gewaschen und die Xylollösung wird abgesaugt. Anschließend arbeitet man wie in Vergleichsbeispiel 1 angegeben.
Man erhält 691 g (4,3 mol) 2,4-Difluornitrobenzol, entsprechend 53,7 % der Theorie, bezogen auf eingesetztes 2,4-Dichlornitrobenzol und insgesamt 305 g (1,8 mol) nicht umgesetztes 2,4-Dichlornitrobenzol + Fluorchlornitrobenzole (monofluorierte Produkte).

## Patentansprüche

1. Verfahren zur Aufarbeitung von Verbindungen der Formel (1)
AZₓArF_{w}Cl_{(y-w)}R_{z} (1)
enthaltenen Reaktionsgemischen,
worin in Formel (1) Az unabhängig voneinander gleich oder verschieden ist und für einen Rest -F, -Cl, -Br, -NO₂, -CN, -CF₃, -CCl₃, -CHO, -CO(CₙH₂ₙ₊₁), wobei n eine ganze Zahl von 1 bis 10 ist, -COX oder -SO₂X steht, wobei X für F, Cl oder Br steht, x eine ganze Zahl von 1 bis 3 ist, Ar einen Phenyl-Rest, Pyridyl-Rest oder Naphthylrest bedeutet, w eine ganze Zahl von 1 bis y ist, y eine ganze Zahl von 1 bis 5 bedeutet, R unabhängig voneinander gleich oder verschieden ist und für H, einen geradkettigen oder verzweigten Alkylrest mit 1 bis 10 Kohlenstoffatomen oder einen geradkettigen oder verzweigten Alkoxyrest mit 1 bis 10 Kohlenstoffatomen steht, z eine ganze Zahl von 1 bis 5 ist, wobei (x+y+z) die Zahl aller substituierbarer Valenzen am Rest Ar bedeutet,
dadurch gekennzeichnet, dass man ein durch Umsetzung einer Verbindung der Formel (2)
AzₓArCl_{y}R_{z} (2)
mit einem Alkalimetallfluorid oder einem Gemisch von Alkalimetallfluoriden in Gegenwart einer Komponente a) oder einer Mischung der Komponente a) und wenigstens einer der Komponenten b), c), d) und e) als Katalysator in Anwesenheit oder Abwesenheit eines organischen Lösungsmittels bei 50 bis 250°C hergestelltes Reaktionsgemisch mit Wasser versetzt, Reaktionsgemisch und Wasser in innigen Kontakt bringt und die wässrige Phase abtrennt,
worin in Formel (2) Az, x, Ar, y, R, z und (x + y + z) die gleiche Bedeutung wie in Formel (1) haben
und wobei Komponente a) eine oder mehrere quartäre Ammoniumverbindungen der Formel (3) 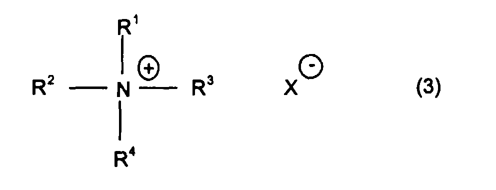 bedeutet,
worin
R¹, R² und R³ gleich oder verschieden sind und einen geradkettigen oder verzweigten Rest der Formel -(CₘH₂ₘO)ₚR⁵ bedeuten, worin R⁵ für Wasserstoff oder einen geradkettigen oder verzweigten Alkylrest mit 1 bis 16 Kohlenstoffatomen steht, m eine ganze Zahl von 1 bis 10 und p eine Zahl von 1 bis 15 bedeuten;
oder einen geradkettigen oder verzweigten Alkylrest mit 1 bis 30 Kohlenstoffatomen;
oder einen unsubstituierten Phenyl- oder Naphthylrest; oder einen substituierten Phenyl- oder Naphthylrest bedeuten, wobei die Substituenten die Bedeutung Halogen, C₁-C₄-Alkyl, C₁-C₄-Alkoxy, Nitro oder Cyano haben;
R⁴ einen geradkettigen oder verzweigten Rest der Formel -(CₘH₂ₘO)ₚR⁵ bedeutet; und
X^{e} ein einwertiger Säurerest oder das Äquivalent eines mehrwertigen Säurerests ist,
die Komponente b) ein oder mehrere Amidophosphoniumsalze der Formel (4) 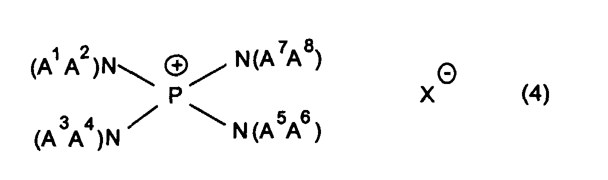 bedeutet,
worin A¹, A², A³, A⁴, A⁵, A⁶, A⁷, A⁸ unabhängig voneinander, gleich oder verschieden sind und für ein geradkettiges oder verzweigtes Alkyl oder Alkenyl mit 1 bis 12 Kohlenstoffatomen, Cycloalkyl mit 4 bis 8 Kohlenstoffatomen, für ein Aryl mit 6 bis 12 Kohlenstoffatomen, ein Aralkyl mit 7 bis 12 Kohlenstoffatomen stehen, oder A¹A², A³A⁴, A⁵A⁶, A⁷A⁸ unabhängig voneinander, gleich oder verschieden sind und direkt oder über O oder N-A⁹ miteinander zu einem Ring mit 3 bis 7 Ringgliedern verbunden sind, A⁹ für ein Alkyl mit 1 bis 4 Kohlenstoffatomen und X⁻ ein einwertiger Säurerest oder das Äquivalent eines mehrwertigen Säurerestes ist, die Komponente c) eine oder mehrere quartäre Ammoniumverbindungen der Formel (5) 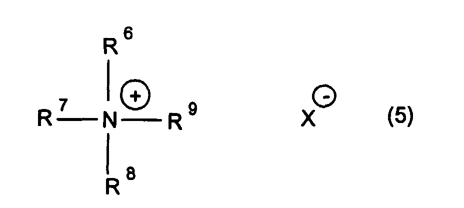 bedeutet,
worin
R⁶, R⁷, R⁸ und R⁹ gleich oder verschieden sind und für einen geradkettigen oder verzweigten Alkylrest mit 1 bis 22 Kohlenstoffatomen; oder für einen unsubstituierten oder substituierten Arylrest oder für einen C₁-C₄-Alkyl-arylrest stehen, wobei Aryl die Bedeutung Phenyl oder Naphthyl hat und die besagten Substituenten Halogen, C₁-C₄-Alkyl, C₁-C₄-Alkoxy, Nitro oder Cyano bedeuten; und X⁻ ein einwertiger Säurerest oder das Äquivalent eines mehrwertigen Säurerests ist;
die Komponente d) eine oder mehrere quartäre Phosphoniumverbindungen der Formel (6) 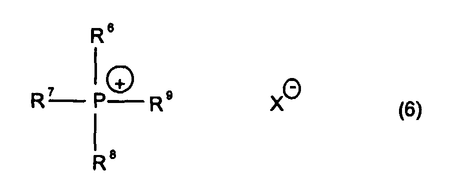 bedeutet,
worin
R⁶, R⁷, R⁸ und R⁹ gleich oder verschieden sind und für einen linearen oder verzweigten Alkylrest mit 1 bis 22 Kohlenstoffatomen; oder für einen unsubstituierten oder substituierten Arylrest oder für einen C₁-C₄-Alkyl-arylrest stehen, wobei Aryl die Bedeutung Phenyl oder Naphthyl hat und die besagten Substituenten Halogen, C₁-C₄-Alkyl, C₁-C₄-Alkoxy, Nitro oder Cyano bedeuten; und X⁻ ein einwertiger Säurerest oder das Äquivalent eines mehrwertigen Säurerests ist;
die Komponente e) einen oder mehrere Ether der Formel (7)
R¹⁰-(O-CₐH₂ₐ)_{b}-OR¹¹ (7)
oder Kronenether bedeutet, wobei in Formel (7)
R¹⁰ und R¹¹ gleich oder verschieden sind und einen geradkettigen oder verzweigten Alkylrest mit 1 bis 16 Kohlenstoffatomen bedeuten;
a eine ganze Zahl von 2 bis 6 und
b eine ganze Zahl von 0 bis 20 ist.

2. Verfahren nach Anspruch 1, dadurch gekennzeichnet, dass man eine Verbindung der Formel (2), worin Az unabhängig voneinander gleich oder verschieden ist und für einen Rest -F, -Cl, -NO₂, -CN, -CF₃, -CCl₃, -CHO oder -CO(CₙH₂ₙ₊₁) steht, wobei n eine ganze Zahl von 1 bis 6 ist, umsetzt.

3. Verfahren nach Anspruch 1 oder 2, dadurch gekennzeichnet, dass man eine Verbindung der Formel (2), worin Az unabhängig voneinander gleich oder verschieden ist und für einen Rest -Cl, -NO₂, -CN, -CF₃ oder -CHO steht, umsetzt.

4. Verfahren nach einem oder mehreren der Ansprüche 1 bis 3, dadurch gekennzeichnet, dass man eine Verbindung der Formel (2), worin Ar für einen Phenylrest steht, umsetzt.

5. Verfahren nach einem oder mehreren der Ansprüche 1 bis 4, dadurch gekennzeichnet, dass man eine Verbindung der Formel (2), worin y eine ganze Zahl von 1 bis 3 ist, umsetzt.

6. Verfahren nach einem oder mehreren der Ansprüche 1 bis 5, dadurch gekennzeichnet, dass man als Alkalimetallfluorid NaF, KF, RbF, CsF oder ein Gemisch derselben einsetzt.

7. Verfahren nach einem oder mehreren der Ansprüche 1 bis 6, dadurch gekennzeichnet, dass man das Reaktionsgemisch bei 20 bis 80°C mit Wasser versetzt, in Kontakt bringt und die wäßrige Phase abtrennt.

8. Verfahren nach einem oder mehreren der Ansprüche 1 bis 7, dadurch gekennzeichnet, dass man das Reaktionsgemisch bei 20 bis 50°C mit Wasser versetzt, in Kontakt bringt und die wäßrige Phase abtrennt.

9. Verfahren nach einem oder mehreren der Ansprüche 1 bis 8, dadurch gekennzeichnet, dass man das Reaktionsgemisch mit einer Menge Wasser versetzt, die ausreicht, die im Reaktionsgemisch vorhandenen wasserlöslichen Bestandteile zu lösen.

10. Verfahren nach einem oder mehreren der Ansprüche 1 bis 9, dadurch gekennzeichnet, dass man das Reaktionsgemisch mit einer Menge Wasser versetzt, die ausreicht, die im Reaktionsgemisch vorhandenen Alkalimetallsalze zu lösen.
